# EUROPEAN PATENT APPLICATION

(11) **EP 2 873 738 A1**
(43) Date of publication of application: **20.05.2015**
(21) Application number: 13192992.9
(22) Date of filing: 15.11.2013
(51) Int. Cl.: C12Q 1/68

(54) **SNP composition and method for diagnosing risk for dyslipidemia**

(71) Applicant: Latvian Biomedical Research and Study Centre, 1067 Riga (LV)
(72) Inventor: Radovica, Ilze, 1067 Riga (LV); Fridmanis, Davids, 1067 Riga (LV); Nikitina-Zake, Liene, 1067 Riga (LV); Klovins, Janis, 1067 Riga (LV)
(74) Representative: Kuzjukevica, Lucija

(57) **Abstract**

A method for predicting in a subject a level of blood LDL-C linked with dyslipidemia comprising detecting the presence of composition of common single nucleotide polymorphism (SNP) in a target polynucleotide in a subject, wherein the SNP comprises rs1167998, rs10889353, rs541041, rs754524, rs949790, rs10474433, rs3846662, rs1883025, rs662799, rs2271293, rs2965101, rs4803750, rs157580, rs2075650 and rs405509, and wherein detecting presence of the SNP in the subject is indicative of a propensity or increased risk of developing a dyslipidemia.

SEQUENCE LISTING

<110> Latvian Biomedical Research and Study Centre

<120> SNP composition and method for diagnosing risk of dyslipidemia

<130> AK/P2782

<160> 15

<170> BiSSAP 1.2

<210> 1
<211> 52
<212> DNA
<213> Homo sapiens

<220>
<221> source
<222> 1..52
<223> /mol_type="unassigned DNA"
      /organism="Homo sapiens"

<400> 1
tgatttgaga aaaagtgaag tcattacacg acaaggcaaa gggaaggtaa ag          52


<210> 2
<211> 52
<212> DNA
<213> Homo sapiens

<220>
<221> source
<222> 1..52
<223> /mol_type="unassigned DNA"
      /organism="Homo sapiens"

<400> 2
gagcctgagc caccttatct gttaaaccct taaatgagat gagtgcaaag tg          52


<210> 3
<211> 52
<212> DNA
<213> Homo sapiens

<220>
<221> source
<222> 1..52
<223> /mol_type="unassigned DNA"
      /organism="Homo sapiens"

<400> 3
gttacctatt catgtttcag ggcccatatc taatgccact tactcttact gc          52


<210> 4
<211> 52
<212> DNA
<213> Homo sapiens

<220>

<221> source
<222> 1..52
<223> /mol_type="unassigned DNA"
      /organism="Homo sapiens"

<400> 4
tctaagcctt aagttatttt ttaagtctat gaatgtatag cttgttttct aa          52


<210> 5
<211> 52
<212> DNA
<213> Homo sapiens

<220>
<221> source
<222> 1..52
<223> /mol_type="unassigned DNA"
      /organism="Homo sapiens"

<400> 5
tttgaaagag tcagacacaa atgcaagcat accatatgct ctactgcact tt          52


<210> 6
<211> 52
<212> DNA
<213> Homo sapiens

<220>
<221> source
<222> 1..52
<223> /mol_type="unassigned DNA"
      /organism="Homo sapiens"

<400> 6
tgtaactctt tatccatagc tgtcattctt ggtctctctg aagcatgctc tc          52


<210> 7
<211> 52
<212> DNA
<213> Homo sapiens

<220>
<221> source
<222> 1..52
<223> /mol_type="unassigned DNA"
      /organism="Homo sapiens"

<400> 7
ccttaaaact cttctcattg ccttacttat gatgtatttt taaactggca aa          52


<210> 8
<211> 52
<212> DNA
<213> Homo sapiens

<220>
<221> source
<222> 1..52
<223> /mol_type="unassigned DNA"

/organism="Homo sapiens"

<400> 8
aggtctctag tgagagaaca gggactggaa tctgtggatc ctatctgttt ta          52

<210> 9
<211> 52
<212> DNA
<213> Homo sapiens

<220>
<221> source
<222> 1..52
<223> /mol_type="unassigned DNA"
      /organism="Homo sapiens"

<400> 9
tgagccccag gaactggagc gaaagtgaga tttgccccat gaggaaaagc tg          52

<210> 10
<211> 52
<212> DNA
<213> Homo sapiens

<220>
<221> source
<222> 1..52
<223> /mol_type="unassigned DNA"
      /organism="Homo sapiens"

<400> 10
agcctgggcc cagggtcaat ggggtagaag atgttgatag tgtaatgttt gg          52

<210> 11
<211> 52
<212> DNA
<213> Homo sapiens

<220>
<221> source
<222> 1..52
<223> /mol_type="unassigned DNA"
      /organism="Homo sapiens"

<400> 11
gagaaggaga ggaattctag aatcctggat ttttaggaac aagggattcc ag          52

<210> 12
<211> 52
<212> DNA
<213> Homo sapiens

<220>
<221> source
<222> 1..52
<223> /mol_type="unassigned DNA"
      /organism="Homo sapiens"

<400> 12

cagcatgttg aattctattt tccccagtga agcaggtcct gctattgaaa ac          52

<210> 13
<211> 52
<212> DNA
<213> Homo sapiens

<220>
<221> source
<222> 1..52
<223> /mol_type="unassigned DNA"
      /organism="Homo sapiens"

<400> 13
ggtcacggtg tcagcaaggt gtcagcgagg ttccttgggt atgggaccca aa          52

<210> 14
<211> 52
<212> DNA
<213> Homo sapiens

<220>
<221> source
<222> 1..52
<223> /mol_type="unassigned DNA"
      /organism="Homo sapiens"

<400> 14
agagatgaga gttggtgtgg ggttggggtg gagtgtgaca gcgtttctct tc          52

<210> 15
<211> 52
<212> DNA
<213> Homo sapiens

<220>
<221> source
<222> 1..52
<223> /mol_type="unassigned DNA"
      /organism="Homo sapiens"

<400> 15
gaagggagga cacctcgccc agtaatccag acaccctcct ccattctggg gg          52

## Description

The present invention relates to a composition and method of common single nucleotide polymorphisms for predicting risk of dyslipidemia via "optimal LDL-C level" or "near optimal LDL-C level", "borderline LDL-C level" and "high LDL-C level" or "increased LDL-C level" in an individual.

Dyslipidemia is a disorder of lipoprotein metabolism, including lipoprotein overproduction or deficiency. Dyslipidemia may be manifested by elevation of the total cholesterol (TH), the "bad" low-density lipoprotein cholesterol (LDL-C), triglycerides (TG) and a decrease in the "good" high-density lipoprotein cholesterol (HDL-C) concentration in the blood [1]. Dyslipidemias conditions include hyperlipidemia where there is an excess of lipids in the blood or hypolipoproetinaemia where there is a malfunction in the metabolism of lipoproteins resulting in low levels of lipids in the blood. The most common types of dyslipidemia are high cholesterol (called hypercholesterolemia or "hyperlipoproteinemia") and high triglycerides (hypertriglyceridemia)[2].

Clinically, dislipidemia are similar to familial hypercholesterolemia (FH), only the symptoms are milder and more unclear. Dyslipidemia like FH mostly is characterized by elevated levels of LDL-C and TH in the circulation, which deposits of cholesterol in peripheral tissues. Usually dyslipidemia is asymptomatic, but may cause xanthelasmas, and after a prolonged period ischemic heart disease, atherosclerosis which may result in widespread clinical manifestations, including coronary heart disease (CHD), cerebrovascular disease (CVD) and peripheral vascular disease (PVD) or even kidney disease.

Dyslipidemia - a leading risk factor for CHD and stroke includes high LDL-C, low HDL-C, and elevated TG levels. According to a survey conducted in 2003, almost half of US adults had total cholesterol levels of 200 mg/dL or higher. Nearly 40% had borderline high or higher LDL-C levels at or above 130 mg/dL, while more than 20% had HDL-C below the desired threshold of 40 mg/dL [3]. Many subjects with hypercholesterolemia die each year as a result, and many have a reduced quality of life. Inevitably, this places very heavy demands on health service resources.

Because hypercholesterolemia responds well to drug treatment, making an early diagnosis to reduce atherosclerosis risk is beneficial. Treatment for dyslipidemia depends on the condition, but can usually include maintaining a healthy diet, moderate exercise and taking medications [2].

The diagnosis of hypercholesterolemia has traditionally been based on the detection of elevated TH levels in subjects belonging to families with high frequencies of early-onset coronary artery disease (CAD) and/or primary hypercholesterolemia. However, establishing an accurate diagnosis of hypercholesterolemia is still difficult and as such, the disorder is severely under-diagnosed and under-treated in many countries.

Dyslipidemia is caused by several inherited genetic disorders. Risk factors also include obesity, medications, hypothyroidism, a high-fat diet, inactivity, and smoking. Recent genome-wide association studies (GWASs) and other human genetic studies have localized many common single nucleotide polymorphisms (SNPs) and many loci that influence different blood lipid levels including previously known loci that are potentially involved in lipid metabolism [4-23].

Systematic screening for functional mutations in genes associated with FH is also one way of diagnosing hypercholesterolemia. However, it is well known in the art that genetic variation exists among human individuals and in particular between ethnic groups. There are marked physical and physiological differences among human populations that presumably reflect long-term adaptation to unique ecological conditions, random genetic drift, and sex selection. In contemporary populations, these differences are evident both in morphological differences between ethnic groups and in differences in susceptibility and resistance to diseases. There are thus genetic markers that exist in one population and not in others and there are also genetic markers at loci at which different alleles are fixed in different populations. The hypercholesterolemia risk associated specific genetic variants can be substantially different among different ethnic groups. Accordingly, the current method of systemic screening is not universal and equally accurate for all populations.

There is known patent application KR20110094793A describing a kit for analyzing human blood fatty acid composition which consists of SNPs in ADIPOQ gene. International patent application WO2012001613 and European Patent Application 1716171 describe identification of SNPs associated with hyperlipidemia, dyslipidemia and defective carbohydrate metabolism.

Aim of the invention is to provide a method for screening or predicting in a subject a level of blood LDL-C linked with risk of dyslipidemia.

Present invention includes a method for predicting in a subject a level of blood LDL-C linked with dyslipidemia comprising detecting the presence of composition of single nucleotide polymorphisms (SNPs) in a target polynucleotide in a subject. The SNPs comprises rs1167998 (SEQ IN NO. 1), rs10889353 (SEQ IN NO. 2), rs541041 (SEQ IN NO. 3), rs754524 (SEQ IN NO. 4), rs949790 (SEQ IN NO. 5), rs10474433 (SEQ IN NO. 6), rs3846662 (SEQ IN NO. 7), rs1883025 (SEQ IN NO. 8), rs662799 (SEQ IN NO. 9), rs2271293 (SEQ IN NO. 10), rs2965101 (SEQ IN NO. 11), rs4803750 (SEQ IN NO. 12), rs157580 (SEQ IN NO. 13), rs2075650 (SEQ IN NO. 14) and/or rs405509 (SEQ IN NO. 15). Detecting a presence of the SNPs in the subject is indicative of a propensity or increased risk of developing a dyslipidemia. The presence of the SNPs is indicative of a subject with optimal LDL-C level, near optimal LDL-C level or high LDL-C level.

Said invention also includes detecting the presence of a single nucleotide polymorphisms (SNPs) at a position rs1167998 (nucleotide 27 on SEQ ID NO.1) or any of rs10889353 (nucleotide 27 on SEQ ID NO. 2), rs541041 nucleotide 27 on SEQ ID NO. 3, rs754524 (nucleotide 27 on SEQ ID NO. 4), rs949790 (nucleotide 27 on SEQ ID NO. 5), rs10474433 (nucleotide 27 on SEQ ID NO. 6), rs3846662 (nucleotide 27 on SEQ ID NO. 7), rs1883025 (nucleotide 27 on SEQ ID NO. 8), rs662799 (nucleotide 27 On SEQ ID NO. 9), rs2271293 (nucleotide 27 on SEQ ID NO. 10), rs2965101 (nucleotide 27 on SEQ ID NO. 11), rs4803750 (nucleotide 27 On SEQ ID NO. 12), rs157580 (nucleotide 27 on SEQ ID NO. 13), rs2075650 (nucleotide 27 on SEQ ID NO. 14) or rs405509 (nucleotide 27 on SEQ ID NO. 15).

A probe for the diagnosing of dyslipidemia comprising composition of single nucleotide polymorphisms (SNPs) set forth as rs1167998 (SEQ IN NO. 1), rs10889353 (SEQ IN NO. 2), rs541041 (SEQ IN NO. 3), rs754524 (SEQ IN NO. 4), rs949790 (SEQ IN NO. 5), rs10474433 (SEQ IN NO. 6), rs3846662 (SEQ IN NO. 7), rs1883025 (SEQ IN NO. 8), rs662799 (SEQ IN NO. 9), rs2271293 (SEQ IN NO. 10), rs2965101 (SEQ IN NO. 11), rs4803750 (SEQ IN NO. 12), rs157580 (SEQ IN NO. 13), rs2075650 (SEQ IN NO. 14) and/or rs405509 (SEQ IN NO. 15). Said probes can be implemented also as a kit including additional elements for example fluorescents for ease of detection of said SNPs. In another embodiment a probe can comprise composition of SNPs set for the as rs1167998 (nucleotide 27 on SEQ ID NO.1) or any of rs10889353 (nucleotide 27 on SEQ ID NO. 2), rs541041 nucleotide 27 on SEQ ID NO. 3, rs754524 (nucleotide 27 on SEQ ID NO. 4), rs949790 (nucleotide 27 on SEQ ID NO. 5), rs10474433 (nucleotide 27 on SEQ ID NO. 6), rs3846662 (nucleotide 27 on SEQ ID NO. 7), rs1883025 (nucleotide 27 on SEQ ID NO. 8), rs662799 (nucleotide 27 On SEQ ID NO. 9), rs2271293 (nucleotide 27 on SEQ ID NO. 10), rs2965101 (nucleotide 27 on SEQ ID NO. 11), rs4803750 (nucleotide 27 On SEQ ID NO. 12), rs157580 (nucleotide 27 on SEQ ID NO. 13), rs2075650 (nucleotide 27 on SEQ ID NO. 14) or rs405509 (nucleotide 27 on SEQ ID NO. 15).

According to one embodiment, the present invention relates to a method for predicting blood LDL-C level in an individual, the method comprise detecting specific genetic variation pattern (SNPs pattern) in certain genes associated with LDL-C metabolism in a sample of the individual. In particular, the genes are ABCA1, ANGPTL3, APOA1, APOB, APOE, DOCK7, HMGCR, LCAT and TOMM40. More in particular, the SNPs are selected from the group of loci with nucleotide base change consisting of: CHR1, g. 62931632 A>C (SEQ ID NO. 1), CHR1, g. 63118196 A>C (SEQ ID NO. 2), CHR2, g. 21294975 C>T (SEQ ID NO. 3), CHR2, g. 21311541 A>C (SEQ ID NO. 4), CHR2, g. 21449987 A>G (SEQ ID NO. 5), CHR5, g. 74616843 C>T (SEQ ID NO. 6), CHR5, g. 74651084 C>T (SEQ ID NO. 7), CHR9, g. 107664301 A>G (SEQ ID NO. 8), CHR11, g. 116663707 A>G (SEQ ID. NO. 9), CHR16, g. 67902070 A>G (SEQ ID NO. 10), CHR19, g. 45237812 A>G (SEQ ID NO. 11), CHR19, g. 45247627 A>G (SEQ ID NO. 12), CHR19, g. 45395266 A>G (SEQ ID NO. 13), CHR19, g. 45395619 A>G (SEQ ID NO. 14), CHR19, g. 45408836 A>C (SEQ ID NO. 15), where "g" stands for genomic sequence and "CHR" stands for number of chromosome. Even more in particular, the SNPs may be selected from the variations listed in TABLE 1.

**TABLE 1**

| List of SNPs | | | | |
|---|---|---|---|---|
| **SNP** | **rs number** | **Sequence** | **Risk Alele** | **SEQ ID NO.** |
| CHR1, g.62931632A>C | rs1167998 | TGATTTGAGAAAAAGTGAAGTCATTA[A/C] ACGACAAGGCAAAGGGAAGGTAAAG | A | 1 |
| CHR1, g.63118196A>C | rs10889353 | GAGCCTGAGCCACCTTATCTGTTAAA[A/C] CCTTAAATGAGATGAGTGCAAAGTG | A | 2 |
| CHR2, g.21294975C>T | rs541041 | GTTACCTATTCATGTTTCAGGGCCCA[C/T] ATCTAATGCCACTTACTCTTACTGC | T | 3 |
| CHR2, g.21311541A>C | rs754524 | TCTAAGCCTTAAGTTATTTTTTAAGT[A/C] TATGAATGTATAGCTTGTTTTCTAA | C | 4 |
| CHR2, g.21449987A>G | rs949790 | TTTGAAAGAGTCAGACACAAATGCAA[A/G] CATACCATATGCTCTACTGCACTTT | A | 5 |
| CHR5, g.74616843C>T | rs10474433 | TGTAACTCTTTATCCATAGCTGTCAT[C/T] CTTGGTCTCTCTGAAGCATGCTCTC | C | 6 |
| CHR5, g.74651084C>T | rs3846662 | CCTTAAAACTCTTCTCATTGCCTTAC[C/T] TATGATGTATTTTTAAACTGGCAAA | C | 7 |
| CHR9, g.107664301A>G | rs1883025 | AGGTCTCTAGTGAGAGAACAGGGACT[A/G] GAATCTGTGGATCCTATCTGTTTTA | G | 8 |
| CHR11, g.116663707A>G | rs662799 | TGAGCCCCAGGAACTGGAGCGAAAGT[A/G] AGATTTGCCCCATGAGGAAAAGCTG | G | 9 |
| CHR16, g.67902070A>G | rs2271293 | AGCCTGGGCCCAGGGTCAATGGGGTA[A/G] AAGATGTTGATAGTGTAATGTTTGG | A | 10 |
| CHR19, g.45237812A>G | rs2965101 | GAGAAGGAGAGGAATTCTAGAATCCT[A/G] GATTTTTAGGAACAAGGATTCCAG | A | 11 |
| CHR19, g.45247627A>G | rs4803750 | CAGCATGTTGAATTCTATTTCCCCA[A/G] TGAAGCAGGTCCTGCTATTGAAAAC | A | 12 |
| CHR19, g.45395266A>G | rs157580 | GGTCACGGTGTCAGCAAGGTGTCAGC[A/G] AGGTTCCTTGGGTATGGACCCAAA | A | 13 |
| CHR19, g.45395619A>G | rs2075650 | AGAGATGAGAGTTGGTGTGGGGTTGG[A/G] GTGGAGTGTGACAGCGTTTCTCTTC | G | 14 |
| CHR19, g.45408836A>C | rs405509 | GAAGGGAGGACACCTCGCCCAGTAAT[A/C] CAGACACCCTCCTCCATTCTGGGGG | A | 15 |

The present invention also provides microarrays, chips, and/or kits comprising at least one probe capable of hybridizing to at least one SNP according to any aspect of the present invention.

Definitions for convenience, certain terms employed in the specification, examples and appended claims are collected here.

The term "comprising" is herein defined to be that where the various components, ingredients, or steps, can be conjointly employed in practicing the present invention. Accordingly, the term "comprising" encompasses the more restrictive terms "consisting essentially of" and "consisting of." With the term "consisting essentially of" it is understood that the method according to any aspect of the present invention "substantially" comprises the indicated SNP as "essential" element. Additional SNPs may be included. Accordingly, a method "consisting essentially of" a plurality of SNPs will be novel in view of a known polypeptide accidentally comprising one of the SNPs. With the term "consisting of" it is understood that the method, microarray and/or chip according to the invention corresponds to all of the SNPs.

The term "label" or "label containing moiety" refers in a moiety capable of detection, such as a radioactive isotope or group containing same and nonisotopic labels, such as enzymes, biotin, avidin, streptavidin, digoxygenin, luminescent agents, dyes, haptens, and the like. Luminescent agents, depending upon the source of exciting energy, can be classified as radio luminescent, chemiluminescent, bio luminescent, and photo luminescent (including fluorescent and phosphorescent). A probe described herein can be bound, for example, chemically bound to label-containing moieties or can be suitable to be so bound. The probe can be directly or indirectly labelled.

The term "locus" is herein defined to be a specific location of a gene or DNA sequence on a chromosome. A variant of the DNA sequence at a given locus is called an allele. The ordered list of loci known for a particular genome is called a genetic map. Gene mapping is the process of determining the locus for a particular biological trait.

The term "polymorphism" is herein defined to be the occurrence of genetic variations that account for alternative DNA sequences and/or alleles among individuals in a population.

The term "polymorphic site" is herein defined to be a genetic locus wherein one or more particular sequence variations occur. A polymorphic site can be one or more base pairs. For example, a "single nucleotide polymorphism (SNP)" is a polymorphism that occurs at a single nucleotide. As used herein, a "cluster" of SNPs refers to three or more SNPs that occur within 100 kilobases of each other in a particular polymorphic site, wherein all of the SNPs have a p-value e<"4> (i.e. < 1 x 10<"4>).

The term "probe" is herein defined to be an oligonucleotide. A probe can be single stranded at the time of hybridization to a target. Probes include but are not limited to primers, i.e., oligonucleotides that can be used to prime a reaction, for example at least in a PCR reaction. The term "reference nucleotide sequence" used interchangeably with the term "reference sequence" is herein defined to be for a nucleotide sequence of a particular gene for example, in NCBI databases.

Alleles that differ from the reference are referred to as "variant" alleles. The polypeptide encoded by the reference nucleotide sequence is the "reference" polypeptide with a particular reference amino acid sequence, and polypeptides encoded by variant alleles are referred to as "variant" polypeptides with variant amino acid sequences. Nucleotide sequence variants can result in changes affecting properties of a polypeptide. These sequence differences, when compared to a reference nucleotide sequence, include insertions, deletions, conversions and substitutions: e.g. an insertion, a deletion or a conversion may result in a frame shift generating an altered polypeptide; a substitution of at least one nucleotide may result in a premature stop codon, amino acid change or abnormal mRNA splicing; the deletion of several nucleotides, resulting in a deletion of one or more amino acids encoded by the nucleotides; the insertion of several nucleotides, such as by unequal recombination or gene conversion, resulting in an interruption of the coding sequence of a reading frame; duplication of all or a part of a sequence; transposition; or a rearrangement of a nucleotide sequence.

The term "allele" is herein defined to one of a number of alternative forms of the same gene or same genetic locus. It is the alternative form of a gene for a character producing different effects. Sometimes, different alleles can result in different phenotypic traits.

The Term "risk allele" is herein defined to one of an alternative forms of the same gene or same genetic locus that is associated to specific phenotype, for example increased LDL-C level.

The term "sample" is herein defined to include but is not limited to be blood, sputum, saliva, mucosal scraping, tissue biopsy and the like.

Specific polymorphisms in ABCA1, ANGPTL3, APOA1, APOB, APOE, DOCK7, HMGCR, LCAT and TOMM40 gene loci may be used in prediction of LDL-C level in plasma. In particular, these polymorphisms may be SNPs. More in particular, out of these hundreds of SNPs known in the art only 15 may be related to prediction of LDL-C level in plasma.

There is thus a need to provide a method of diagnosing LDL-C level. In particular, there is a need to determine polymorphisms in genes that contribute to the genetic predisposition for LDL-C level. More in particular, the SNPs are selected from the group consisting of: CHR1, g. 62931632 A>C (SEQ ID NO. 1), CHR1, g. 63118196 A>C (SEQ ID NO. 2), CHR2, g. 21294975 C>T (SEQ ID NO. 3), CHR2, g. 21311541 A>C (SEQ ID NO. 4), CHR2, g. 21449987 A>G (SEQ ID NO. 5), CHR5, g. 74616843 C>T (SEQ ID NO. 6), CHR5, g. 74651084 C>T (SEQ ID NO. 7), CHR9, g. 107664301 A>G (SEQ ID NO. 8), CHR11, g. 116663707 A>G (SEQ ID NO. 9), CHR16, g. 67902070 A>G (SEQ ID NO. 10), CHR19, g. 45237812 A>G (SEQ ID NO. 11), CHR19, g. 45247627 A>G (SEQ ID NO. 12), CHR19, g. 45395266 A>G (SEQ ID NO. 13), CHR19, g. 45395619 A>G (SEQ ID NO. 14), CHR19, g. 45408836 A>C (SEQ ID NO. 15). Even more in particular, the SNPs may be selected from the SNPs listed in TABLE 1.

LDL-C level can result from variation in combination of several genes associated with Lipid metabolism. In particular, LDL-C level can result from variation in combination of the ATP-binding cassette, subfamily A, member 1 gene (ABCA1), the Angiopoietin-like 3 gene (ANGPTL3), the Apolipoprotein A-1 gene (APOA1), the Apolipoprotein B gene (APOB), the Apolipoprotein E gene (APOE), the Dedicator of cytokinesis 7 gene (DOCK7), the 3-Hydroxy-3-methilglutaryl-CoA reductase (HMGCR), the Lecitin:cholesterol acyltransferase gene (LCAT) and the Translocase of outer mitochondrial membrane 40 dene (TOMM40) and the like.

In particular, the method comprises detecting the presence of combination of 15 SNPs in the genes as described in a sample of the individual. In particular, the SNPs are: CHR1, g. 62931632 A>C (SEQ ID NO. 1), CHR1, g. 63118196 A>C (SEQ ID NO. 2), CHR2, g. 21294975 C>T (SEQ ID NO. 3), CHR2, g. 21311541 A>C (SEQ ID NO. 4), CHR2, g. 21449987 A>G (SEQ ID NO. 5), CHR5, g. 74616843 C>T (SEQ ID NO. 6), CHR5, g. 74651084 C>T (SEQ ID NO. 7), CHR9, g. 107664301 A>G (SEQ ID NO. 8), CHR11, g. 116663707 A>G (SEQ ID NO. 9), CHR16, g. 67902070 A>G (SEQ ID NO. 10), CHR19, g. 45237812 A>G (SEQ ID NO. 11), CHR19, g. 45247627 A>G (SEQ ID NO. 12), CHR19, g. 45395266 A>G (SEQ ID NO. 13), CHR19, g. 45395619 A>G (SEQ ID NO. 14), CHR19, g. 45408836 A>C (SEQ ID NO. 15). More in particular, the SNP are listed in Table 1.

A list of SNPs that are used to predict LDL-C level in an individual. The method comprises detecting all SNPs in the genes as described in a sample of the individual. In the genetic context, combinations of more than 23 risk alleles from 15 SNPs in ABCA1, ANGPTL3, APOA1, APOB, APOE, DOCK7, HMGCR, LCAT and TOMM40 gene locus, results in increased level of LDL-C in plasma. The level of plasma LDL-C in individuals with 22 and less risk alleles from 15 SNPs in previously mentioned genes is on the borderline or near optimal.

According to any aspect of the present invention, the method for predicting LDL-C level in plasma in an individual, consisting of or consisting essentially of detecting the presence of 15 SNPs in all gene loci disclosed. More in particular, the method involves the detection of 15 SNPs in all gene loci according to any aspect of the present invention.

According to another aspect, there is provided at least one method for predicting optimal LDL-C level in plasma in an individual, the method comprising detecting the presence of 10 or less risk alleles in all 15 SNPs in all gene loci in an individual.

The SNPs may be determined by a microarray analysis. This is advantageous as it is efficient and more accurate than the methods known in the art. The method according to any aspect of the present invention may further comprise a step of correlating results of the detection of SNPs with one or more clinicopathological data to implement a particular treatment plan for the individual. In particular, the clinicopathological data may be selected from the group consisting of the individual's age, lifestyle, previous personal and/or familial history of increased LDL-C level in plasma, previous personal and/or familial history of response to medications, any genetic or biochemical predisposition to increased LDL-C level in plasma and the like.

According to another embodiment, there is provided a microarray and/or DNA chip comprising, consisting of or consisting essentially of probes capable of hybridizing to 15 SNPs of ABCA1, ANGPTL3, APOA1, APOB, APOE, DOCK7, HMGCR, LCAT and TOMM40 gene loci according to any aspect of the present invention in a sample nucleic acid of an individual, wherein the SNPs are selected from the group consisting of: CHR1, g. 62931632 A>C (SEQ ID NO. 1), CHR1, g. 63118196 A>C (SEQ ID NO. 2), CHR2, g. 21294975 C>T (SEQ ID NO. 3), CHR2, g. 21311541 A>C (SEQ ID NO. 4), CHR2, g. 21449987 A>G (SEQ ID NO. 5), CHR5, g. 74616843 C>T (SEQ ID NO. 6), CHR5, g. 74651084 C>T (SEQ ID NO. 7), CHR9, g. 107664301 A>G (SEQ ID NO. 8), CHR11, g. 116663707 A>G (SEQ ID NO. 9), CHR16, g. 67902070 A>G (SEQ ID NO. 10), CHR19, g. 45237812 A>G (SEQ ID NO. 11), CHR19, g. 45247627 A>G (SEQ ID NO. 12), CHR19, g. 45395266 A>G (SEQ ID NO. 13), CHR19, g. 45395619 A>G (SEQ ID NO. 14), CHR19, g. 45408836 A>C (SEQ ID NO. 15) and the like. More in particular, the SNPs may be SNPs selected from TABLE 1.

The probes capable of hybridizing to all SNPs of ABCA1, ANGPTL3, APOA1, APOB, APOE, DOCK7, HMGCR, LCAT and TOMM40 gene loci according to any aspect of the present invention in a sample nucleic acid of an individual are designed using any one of SEQ ID NO.1 - 15 in TABLE 1.

According to a further aspect of the invention, there is provided a kit for determining whether an individual has an increased risk for increased LDL-C level, the kit comprising: (a) at least 15 oligonucleotides that can identify an LDL-C level associated SNPs in all gene loci according to any aspect of the present invention wherein the SNPs may be selected from the group consisting of: CHR1, g. 62931632 A>C (SEQ ID NO. 1), CHR1, g. 63118196 A>C (SEQ ID NO. 2), CHR2, g. 21294975 C>T (SEQ ID NO. 3), CHR2, g. 21311541 A>C (SEQ ID NO. 4), CHR2, g. 21449987 A>G (SEQ ID NO. 5), CHR5, g. 74616843 C>T (SEQ ID NO. 6), CHR5, g. 74651084 C>T (SEQ ID NO. 7), CHR9, g. 107664301 A>G (SEQ ID NO. 8), CHR11, g. 116663707 A>G (SEQ ID NO. 9), CHR16, g. 67902070 A>G (SEQ ID NO. 10), CHR19, g. 45237812 A>G (SEQ ID NO. 11), CHR19, g. 45247627 A>G (SEQ ID NO. 12), CHR19, g. 45395266 A>G (SEQ ID NO. 13), CHR19, g. 45395619 A>G (SEQ ID NO. 14), CHR19, g. 45408836 A>C (SEQ ID NO. 15) and the like; and (b) instructions for use. More in particular, the SNP are selected from the SNPs listed in TABLE 1.

Having now generally described the invention, the same will be more readily understood through reference to the following examples which are provided by way of illustration, and are not intended to be limiting of the present invention.

A person skilled in the art will appreciate that the present invention may be practised without undue experimentation according to the method given herein. The methods, techniques and chemicals are as described in the references given or from protocols in standard biotechnology and molecular biology text books.

Standard molecular biology techniques known in the art and not specifically described were generally followed as described in manufacturer's instructions (Illumina Golden Gate genotyping assay).

### Study population and data collection

The study included 1273 subjects recruited between September 2003 and September 2011 from the Genome Database of Latvian Population (LGDB). A total of 853 (67%) females and 420 (33%) males, were recruited from a cross-sectional, genetically unrelated population of Latvia. Semi-structured questionnaires were used to collect the demography, clinical data and family history of participants. All subjects undertook a screening protocol consisting of clinical history and laboratory tests including blood serum lipids. Blood samples were analyzed for the above tests using standard techniques. Written informed consent was acquired from all LGDB participants before their inclusion in the register. The study protocol was approved by the Central Medical Ethics Committee of Latvia (Protocols Nr. 2007 A-7 and 01-29.1/25). The characteristics of the study subjects are detailed in TABLE 2.

**TABLE 2**

| Details of study subjects | |
|---|---|
| Variable | |
| n | 1273 |
| SNPs genotyped | 139 |
| Genotyping rate, % | 99.76 % |
| Mean age, years ± SD (min-max) | 52.05 ± 13.73 (18-82) |
| Female gender, % | 67.0 % |
| TH-level, mmol/L ± SD (min-max) | 5.78 ± 1.24 (1.95-11.7) |
| LDL-level, mmol/L ± SD (min-max) | 3.57 ± 1.07 (0.72-8.4) |
| HDL-level, mmol/L ± SD (min-max) | 1.59 ± 0.44 (0.33-3.52) |
| TG-level, mmol/L ± SD (min-max) | 1.40 ± 0.75 (0.29-5.87) |
| LogTG, mmol/L ± SD (min-max) | 0.1 ± 0.21 (-0.54-0.77) |
| BMI, kg/m² ± SD (min-max) | 27.02 ± 5.14 (15.64-59.73) |

### DNA isolation and normalization

DNA was isolated from blood lymphocytes using the standard phenol-chloroform extraction method [24]. Qualitative and quantitative estimations were carried out on the DNA samples. All DNA samples were normalized to 50 ng/µl concentration.

### Genotyping assay design

Selection of SNPs was based on information obtained from the number of candidate gene association and GWA studies [4-23]. SNPs were ranked based on their reported highest P values.

Genotyping assay design, fabrication and quality controls for the genotyping of polymorphic alleles - 144-custom probes were synthesized by *lllumina*®*.* Probes of the SNPs were sent to *lllumina's*® *Illumina Assay Designe Tool* for scoring, *lllumina*® ranked SNPs based on an in-built algorithm in the *Illumina*® *Assay Designe Tool* page where SNPs scoring below 0.4 were given a designability rank of zero thus the probe was not designable by *lllumina*®*.* A score between 0.4 to 0.6 gave a designability rank of 0.5 while a score above 0.6 was given a designability rank of 1 and both these ranks could be successfully designed as probes (*Oligo Pool All, OPA)* by *Illumina*®*.* Probes were submitted to *Illumina*® *Assay Design Tool* in the rs number format.

191 SNPs were excluded from the list based on designability rank of 0. Top 133 SNPs from the resulting list of SNPs was choosen for genotyping resulting in 3.49x10⁻⁷ as the cut-off P value in order to cover the all SNPs. In addition 11 tag-SNPs were included from the GP109A/GPR109B locus using *Haploview software* and *HapMap* data resulting in total number of 144 SNPs representing 32 gene loci. The final number of SNPs corresponds to the requirements of the *Custom VeraCode GoldenGate Genotyping* platform offering estimation of 144 SNPs in a single well of a 96-well microplate as one of the standard options. List of SNPs are shown in TABLE 1.

### Genotyping

Genotyping was performed using the *Illumina® BeadExpress* system *(Illumina® Golden Gate genotyping assay)* which was capable of multiplexing 144 SNPs in a single reaction. All assays were performed on 96- well plates according to the manufacturer's protocol.

The *lllumina*® *GoldenGate genotyping Assay* queried genomic DNA with three oligonucleotide probes for each locus and creates DNA fragments that could be amplified by standard PCR methods using universal primers. For each of the 144 loci interrogated in each assay, the oligo mix contained 2 allele specific and one locus specific probe (i.e. 3 x 144 oligos). The 3' ends of the two alternative allele specific probes were complementary to two universal primers, U1 and U2, with the 5' end complementary to the 3' end of the locus. Each probe sequence terminated at the SNP that is to be assayed with an allele specific base. The third probe, the locus specific probe, was complementary to the genomic DNA that started 5 to 20 bases 3' of the locus in question. As well as a locus specific sequence, this probe also contained a specific *lllumicode* sequence that was used to identify the locus (on the *BeadArray*) as well as the sequence for universal primer sequence U3. All 144 probes was annealed to the genomic DNA at the same time, DNA polymerase was added to close the gap between the allele specific (including either U1 OR U2) and the locus specific (including U3) probes and the paired fragments were ligated together. The probe fragments were then separated from the genomic DNA and used to inoculate a PCR reaction. The primer mix for this PCR reaction comprised primers U1 and U2 labelled with Cy3 and Cy5 respectively and biotinylated primer U3. Any specific GoldenGate assay required a particular pool of oligonucleotides corresponding to the allele and locus specific probes for the loci that will be interrogated. Any given oligo pool could interrogate up to 144 different SNP loci. Multiple oligo pools could be run on sample sets to increase the number of loci queried. Oligo pools were shipped with their own information file designating *lllumicode* which was used to interrogate each locus as well as which allele was labelled with Cy3 and which with Cy5.

Bead scanning and quantification Bead Array reader scanned the hybridized beads and determined the signal intensities for each dye at each bead location.

Custom software, the *Genome Studio* from *lllumina*® used the information filed from the Assay and the oligo pool to map the known location of each allele in the sample back to the locus being interrogated by that code and to match the dye intensities to the specific alleles. The dye intensities were examined by the software to determine the genotype of each sample for that locus. A locus predominantly returning a signal from Cy3 was AA, Cy5 was BB and an even ratio represents a heterozygous individual. Data was returned with the allele call for each locus as well as a so called a Gentrain score, a measure that represents the reliability of that genotyping call. Each locus was examined independently to make sure that the assigned genotypes were robust. Although, it was found that loci with high Gentrain scores usually require no manual intervention.

### Data analysis

All the raw intensity data from the custom assays were fed to the *lllumina GenomeStudioTM* to decipher the true allele calls. Overall genotype call rate was 95 % and above with allelic data successfully generated for 97% of the SNPs loci (140 out of 144). All genotyped SNPs were assessed for deviation from Hardy-Weinberg equilibrium using *PLINK v2.050* software. Subsequent statistical analyses were carried out using *PLINK* toolset. *PLINK* toolset was used for linkage disequilibrium, allelic association and linear regression analysis. A level of p < 0.05 was accepted as statistically significant. For each SNP, the beta coefficient was calculated according to additive model for the association between the genotypes and LDL-C level.

144 potential LDL-C level influencing SNPs from 32 gene loci were examined. Four SNPs with low call rate (<100 calls) and one SNP that were devoid of minor alleles were removed. The genotype frequencies of all of remaining 139 SNPs did not deviate from *Hardy-Weinberg* equilibrium (P > 0.05). By setting the predominant genotype of each of the remaining 139 SNPs as the reference, the beta coefficient for each variant genotype was calculated according to additive model. In total, the association of 22 SNPs with LDL-C level were statistically significant (TABLE 3).

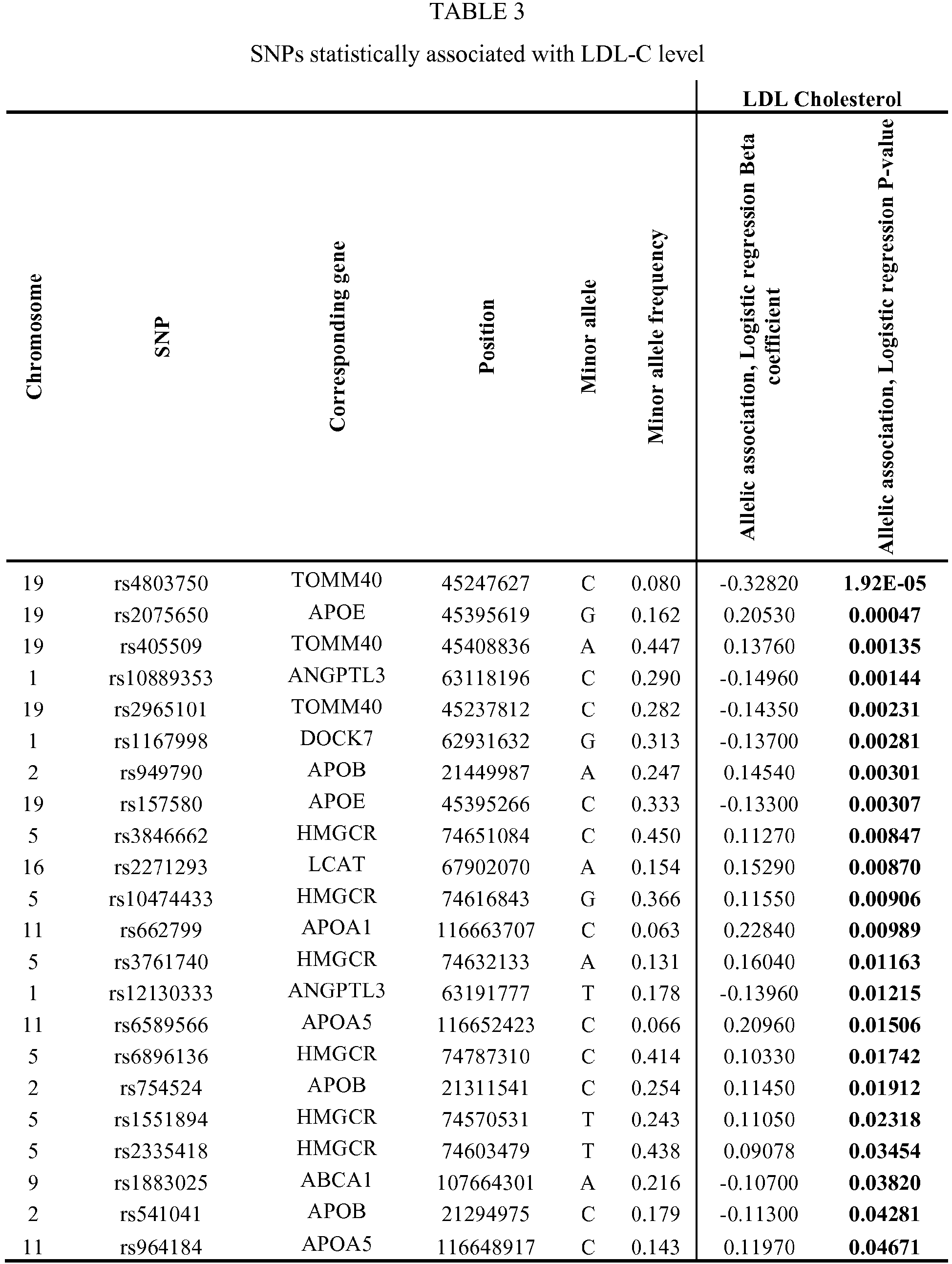

To investigate the effects of risk allele number on blood LDL-C level, risk allele dosage test was performed for SNPs, which were associated with LDL-C level and each represented one haplo block, 15 SNPs representing ABCA1, ANGPTL3, APOA1, APOB, APOE, DOCK7, HMGCR, LCAT and TOMM40 gene loci. All samples were divided into three groups based on the range of risk allele distribution in all possible combinations. All genotyping data for 15 SNPs was divided into three groups based on the number of risk alleles in individual to develop LDL-C level predicting algorithm. Different three-group divisions was made to get the best division for predicting LDL-C level. Risk allele doze analysis was performed to clarify the best division in three groups of risk allele distribution. One sample T-Tests was performed within SPSS software to calculate differences between all these three groups for all possible three-group divisions.

The best tree-group combination to predict LDL-C level was: first group - individuals with 10 and less risk alleles predicts "optimal LDL-C level" or "near optimal LDL-C level"; second group - individuals with 11 to 20 risk alleles predicts "borderline LDL-C level"; and third group - individuals with 21 and more risk alleles predicts "high LDL-C level" or "increased LDL-C level". Results for the best three-group division is indicated in TABLE 4.

**TABLE 4**

| Best three-group division for predicting LDL-C level | | | | |
|---|---|---|---|---|
| **Group name** | **Risk allele range** | **Mean LDL-C level (mmol/L)** | **95% Confidence Interval of the Difference** | |
| | | | **Lower** | **Upper** |
| "optimal LDL-C level" | 10 and less | 2.96 | 2.741 | 3.18 |
| or | | | | |
| "near optimal LDL-C level" | | | | |
| "borderline LDL-C level" | 11 to 20 | 3.61 | 3.55 | 3.67 |
| "high LDL-C level" | 21 and more | 4.321 | 3.18 | 5.46 |
| or | | | | |
| "increased LDL-C level" | | | | |

### REFERENCES

1. http://www.medterms.com/.
2. Reilly, K. [cited; Available from: http://cholesterol.about.com/lw/Health-Medicine/Conditions-and-diseases/Dyslipidemia-.htm].
3. Thom, T., et al., Heart disease and stroke statistics--2006 update: a report from the American Heart Association Statistics Committee and Stroke Statistics Subcommittee. Circulation, 2006. 113(6): p. e85-151.
4. Aulchenko, Y.S., et al., Loci influencing lipid levels and coronary heart disease risk in 16 European population cohorts. Nat Genet, 2009. 41(1): p. 47-55.
5. Burkhardt, R., et al., Common SNPs in HMGCR in micronesians and whites associated with LDL-cholesterol levels affect alternative splicing of exon13. Arterioscler Thromb Vase Biol, 2008. 28(11): p. 2078-84.
6. Chasman, D.I., et al., Genetic loci associated with plasma concentration of low-density lipoprotein cholesterol, high-density lipoprotein cholesterol, triglycerides, apolipoprotein A1, and Apolipoprotein B among 6382 white women in genome-wide analysis with replication. Circ Cardiovasc Genet, 2008. 1(1): p. 21-30.
7. Heid, I.M., et al., Genome-wide association analysis of high-density lipoprotein cholesterol in the population-based KORA study sheds new light on intergenic regions. Circ Cardiovasc Genet, 2008. 1(1): p. 10-20.
8. Hiura, Y., et al., Identification of genetic markers associated with high-density lipoprotein-cholesterol by genome-wide screening in a Japanese population: the Suita study. Circ J, 2009. 73(6): p. 1119-26.
9. Igl, W., et al., Modeling of environmental effects in genome-wide association studies identifies SLC2A2 and HP as novel loci influencing serum cholesterol levels. PLoS Genet. 6(1): p.e1000798.
10. Kathiresan, S., et al., Six new loci associated with blood low-density lipoprotein cholesterol, high-density lipoprotein cholesterol or triglycerides in humans. Nat Genet, 2008. 40(2): p. 189-97.
11. Kathiresan, S., et al., Common variants at 30 loci contribute to polygenic dyslipidemia. Nat Genet, 2009. 41(1): p. 56-65.
12. Kooner, J.S., et al., Genome-wide scan identifies variation in MLXIPL associated with plasma triglycerides. Nat Genet, 2008. 40(2): p. 149-51.
13. Ma, L., et al., Genome-wide association analysis of total cholesterol and high-density lipoprotein cholesterol levels using the Framingham heart study data. BMC Med Genet. 11: p. 55.
14. Pollin, T.I., et al., A null mutation in human APOC3 confers a favorable plasma lipid profile and apparent cardioprotection. Science, 2008. 322(5908): p. 1702-5.
15. Ridker, P.M., et al., Polymorphism in the CETP gene region, HDL cholesterol, and risk of future myocardial infarction: Genomewide analysis among 18 245 initially healthy women from the Women's Genome Health Study. Circ Cardiovasc Genet, 2009. 2(1): p. 26-33.
16. Sabatti, C., et al., Genome-wide association analysis of metabolic traits in a birth cohort from a founder population. Nat Genet, 2009. 41(1): p. 35-46.
17. Sandhu, M.S., et al., LDL-cholesterol concentrations: a genome-wide association study. Lancet, 2008. 371(9611): p. 483-91.
18. Saxena, R., et al., Genome-wide association analysis identifies loci for type 2 diabetes and triglyceride levels. Science, 2007. 316(5829): p. 1331-6.
19. Shen, H., et al., Familial defective apolipoprotein B-100 and increased low-density lipoprotein cholesterol and coronary artery calcification in the old order amish. Arch Intern Med. 170(20): p. 1850-5.
20. Suchindran, S., et al., Genome-wide association study of Lp-PLA(2) activity and mass in the Framingham Heart Study. PLoS Genet. 6(4): p. e1000928.
21. Wallace, C., et al., Genome-wide association study identifies genes for biomarkers of cardiovascular disease: serum urate and dyslipidemia. Am J Hum Genet, 2008. 82(1): p. 139-49.
22. Waterworth, D.M., et al., Genetic variants influencing circulating lipid levels and risk of coronary artery disease. Arterioscler Thromb Vasc Biol. 30(11): p. 2264-76.
23. Willer, C.J., et al., Newly identified loci that influence lipid concentrations and risk of coronary artery disease. Nat Genet, 2008. 40(2): p. 161-9.
24. Maniatis, T., E.F. Fritsch, and J. Sambrook, Molecular Cloning. 1982, New York: Cold Spring Harbor Laboratory.

## Claims

1. A method for predicting in a subject a level of blood LDL-C linked with dyslipidemia comprising detecting the presence of composition of single nucleotide polymorphisms (SNPs) in a target polynucleotide in a subject, wherein the SNPs comprises rs1167998 (SEQ IN NO. 1), rs10889353 (SEQ IN NO. 2), rs541041 (SEQ IN NO. 3), rs754524 (SEQ IN NO. 4), rs949790 (SEQ IN NO. 5), rs10474433 (SEQ IN NO. 6), rs3846662 (SEQ IN NO. 7), rs1883025 (SEQ IN NO. 8), rs662799 (SEQ IN NO. 9), rs2271293 (SEQ IN NO. 10), rs2965101 (SEQ IN NO. 11), rs4803750 (SEQ IN NO. 12), rs157580 (SEQ IN NO. 13), rs2075650 (SEQ IN NO. 14) and rs405509 (SEQ IN NO. 15), and wherein detecting presence of the SNPs in the subject is indicative of a propensity or increased risk of developing a dyslipidemia.

2. The method of claim 1 wherein detecting the presence of a single nucleotide polymorphisms (SNPs) at a position rs1167998 (nucleotide 27 on SEQ ID NO.1) or any of rs10889353 (nucleotide 27 on SEQ ID NO. 2), rs541041 nucleotide 27 on SEQ ID NO. 3, rs754524 (nucleotide 27 on SEQ ID NO. 4), rs949790 (nucleotide 27 on SEQ ID NO. 5), rs10474433 (nucleotide 27 on SEQ ID NO. 6), rs3846662 (nucleotide 27 on SEQ ID NO. 7), rs1883025 (nucleotide 27 on SEQ ID NO. 8), rs662799 (nucleotide 27 On SEQ ID NO. 9), rs2271293 (nucleotide 27 on SEQ ID NO. 10), rs2965101 (nucleotide 27 on SEQ ID NO. 11), rs4803750 (nucleotide 27 On SEQ ID NO. 12), rs157580 (nucleotide 27 on SEQ ID NO. 13), rs2075650 (nucleotide 27 on SEQ ID NO. 14) or rs405509 (nucleotide 27 on SEQ ID NO. 15).

3. The method of claim 1 wherein the presence of the SNPs is indicative of a subject with optimal LDL-C level, near optimal LDL-C level and high LDL-C level.

4. Probes for the diagnosing of dyslipidemia comprising composition of single nucleotide polymorphisms (SNPs) set forth as rs1167998 (SEQ IN NO. 1), rs10889353 (SEQ IN NO. 2), rs541041 (SEQ IN NO. 3), rs754524 (SEQ IN NO. 4), rs949790 (SEQ IN NO. 5), rs10474433 (SEQ IN NO. 6), rs3846662 (SEQ IN NO. 7), rs1883025 (SEQ IN NO. 8), rs662799 (SEQ IN NO. 9), rs2271293 (SEQ IN NO. 10), rs2965101 (SEQ IN NO. 11), rs4803750 (SEQ IN NO. 12), rs157580 (SEQ IN NO. 13), rs2075650 (SEQ IN NO. 14) and rs405509 (SEQ IN NO. 15).
